(19) **Europäisches Patentamt / European Patent Office / Office européen des brevets**

(11) **EP 1 339 744 B1**

(12) **EUROPÄISCHE PATENTSCHRIFT**

(45) Veröffentlichungstag und Bekanntmachung des Hinweises auf die Patenterteilung:
**13.01.2010 Patentblatt 2010/02**

(51) Int Cl.:
*C07K 14/51* (2006.01)     *C12N 15/67* (2006.01)
*A61K 38/18* (2006.01)

(21) Anmeldenummer: **01998560.5**

(22) Anmeldetag: **27.11.2001**

(86) Internationale Anmeldenummer:
**PCT/EP2001/013840**

(87) Internationale Veröffentlichungsnummer:
**WO 2002/044203 (06.06.2002 Gazette 2002/23)**

(54) **HERSTELLUNG VON REKOMBINANTEM BMP-2**

PRODUCTION OF RECOMBINANT BMP-2

PRODUCTION DE BMP-2 RECOMBINEE

(84) Benannte Vertragsstaaten:
**AT BE CH CY DE DK ES FI FR GB GR IE IT LI LU MC NL PT SE TR**

(30) Priorität: **29.11.2000 DE 10059336**

(43) Veröffentlichungstag der Anmeldung:
**03.09.2003 Patentblatt 2003/36**

(73) Patentinhaber:
• **Rudolph, Rainer**
  **06120 Halle (DE)**
• **Schwarz, Elisabeth**
  **06120 Halle (DE)**

(72) Erfinder:
• **RUDOLPH, Rainer**
  **06120 Halle (DE)**
• **SCHWARZ, Elisabeth**
  **06120 Halle (DE)**
• **HERR, Gerhard**
  **35578 Wetzlar (DE)**
• **HILLGER, Frank**
  **06846 Dessau (DE)**

(74) Vertreter: **Dey, Michael et al**
**Weickmann & Weickmann**
**Patentanwälte**
**Postfach 86 08 20**
**81635 München (DE)**

(56) Entgegenhaltungen:
WO-A-00/56879     WO-A-97/00886
WO-A-98/55137     US-A- 5 013 649

US-A- 5 674 844

• RUPPERT R ET AL: "HUMAN BONE MORPHOGENETIC PROTEIN 2 CONTAINS A HEPARIN-BINDING SITE WHICH MODIFIES ITS BIOLOGICAL ACTIVITY" EUROPEAN JOURNAL OF BIOCHEMISTRY, Bd. 237, Nr. 1, April 1996 (1996-04), Seiten 295-302, XP000891887 ISSN: 0014-2956
• DATABASE EPODOC [Online] EUROPEAN PATENT OFFICE, THE HAGUE, NL; XP002216822 & CN 1 165 189 A (NO 4 ARMY MEDICAL SCHOOL) 19. November 1997 (1997-11-19)
• RUDOLPH R ET AL: "IN VITRO FOLDING OF INCLUSION BODY PROTEINS" FASEB JOURNAL, Bd. 10, Nr. 1, 1996, Seiten 49-56, XP000605154 ISSN: 0892-6638
• LILIE H. ET AL.: "Advances in refolding of proteins produced in E.coli" CURRENT OPINION IN BIOTECHNOLOGY, Bd. 9, 1998, Seiten 497-501, XP002216689
• GRAY A.M. ET AL.: "Requirement for Activin A and Transforming Growth Factor-beta 1 Pro-Regions in Homodimer Assembly" SCIENCE, Bd. 247, Nr. 4948, 16. März 1990 (1990-03-16), Seiten 1328-1330, XP001109030
• EDER J. ET AL.: "Pro-sequence-assisted protein folding" MOLECULAR MICROBIOLOGY, Bd. 16, Nr. 4, 1995, Seiten 609-614, XP008008992
• HILLGER, FRANK: "Humanes proBMP-2: Rekombinante Expression und Rückfaltung aus Einschlusskörpern" StartDateMarker 1999, EndDateMarker * Seite 56 *

EP 1 339 744 B1

Bemerkungen:
Die Akte enthält technische Angaben, die nach dem Eingang der Anmeldung eingereicht wurden und die nicht in dieser Patentschrift enthalten sind.

Bemerkungen:
Die Akte enthält technische Angaben, die nach dem Eingang der Anmeldung eingereicht wurden und die nicht in dieser Patentschrift enthalten sind.

## Beschreibung

Beschrieben wird ein Verfahren zur Gewinnung von biologisch aktivem drBMP-2 (dr: digit removed) und homologer Proteine durch Naturierung von denaturiertem, biologisch inaktivem proBMP-2 und gegebenenfalls anschließender Abspaltung des Propeptids, wobei bereits die naturierte Proform des Proteins biologisch aktiv ist, pro BMP-2 als neues Produkt und dessen Verwendung zur Herstellung eines Mittels zur Förderung des Knochenwachstums.

WO 98/55137 beschreibt knochenmorphogenetische Proteine (bone morphogenetic proteins BMP) und deren Verwendung beim Knochenwachstum, insbesondere knochenmorphogenetische Fusionsproteine. US 5,013,649 beschreibt gereinigte BMP-2-Proteine sowie deren Herstellung. US 5,674,844 beschreibt die Verabreichung von Morphogenen zur Erhöhung der Knochenmasse beziehungsweise zur Verhinderung von Knochenschwund.

BMPs-2 gehört zur Familie der Bone Morphogenetic Proteins (BMPs). Es handelt sich dabei um Knochenwachstumsfaktoren, deren Klonierung und Charakterisierung erstmals 1988 gelang. Die charakteristische Eigenschaft dieser Proteine ist die Fähigkeit zur Induktion endochondraler Knochenneubildung [Wozney et al., Science 242 (1988), 1528-1534].

Strukturell zählt BMP-2 zusammen mit über 30 weiteren Proteinen zur TGF-β-Superfamilie, welche eine sehr hohe strukturelle Ähnlichkeit untereinander aufweisen, obwohl die Sequenzidentität z.T. nur unter 30 % beträgt [Griffith et al., Proc. Natl. Acad. Sci. USA 93 (1996), 878-883]. Ihr wesentliches Strukturmerkmal ist der sogenannte "Cystin-Knoten". Dieser wird durch vier antiparallele β-Faltblattstränge gebildet, von denen je zwei über Disulfidbindungen miteinander verknüpft sind. Dabei formen zwei Cystine zusammen mit dem Peptidgerüst einen Ring, durch den sich eine dritte Disulfidbrücke erstreckt. β-Faltblätter sind das dominierende Sekundärstrukturelement dieser Proteine. Sie sind hier jedoch länger und stärker verdreht als in anderen β-faltblattreichen Proteinen. Aufgrund der strukturellen Verwandtschaft der Proteine der TGF-β-Familie zueinander haben wir postuliert, dass diese Proteine auch auf ähnliche Weise ihre native Konformation einnehmen, also falten.

BMPs und verwandte Proteine, wie DPP (Decapentaplegic, *Drosophila*), spielen eine wichtige Rolle in der Embryonalentwicklung höherer Organismen. Defekte in BMP- bzw. BMP-Rezeptorgenen oder deren Regulation führen zu z.T. schweren Entwicklungsstörungen, wie z.B. Fibrodysplasia ossificans progressiva [Kaplan et al., Calcif. Tissue Int. 47 (1990), 117-125; Rao et al., Hum. Genet. 90 (1992), 299-302; Shafritz et al., N. Engl. J. Med. 335 (1996), 555-561] und Dentinogenesis imperfecta [Tabas et al., Clin. Orthop. 293 (1993), 310-316]. BMP-Deletionsmutanten der Maus sterben bereits im Embryonalstadium oder kurz nach der Geburt und weisen Fehlbildungen des Skeletts und den verschiedensten Organen, wie Herz, Niere und Augen, auf [Dudley et al., Genes Dev. 9 (1995), 2795-2807; Zhang und Bradley, Development 122 (1996), 2977-2986].

BMPs und ähnliche Wachstumsfaktoren sind für die Therapie systemischer Erkrankungen, wie Osteoporose, nur bedingt geeignet, da sie nicht tolerable Nebenwirkungen, wie beispielweise Tumorpromotion, hervorrufen können. BMPs, insbesondere BMP-2, sind vielmehr für die lokal begrenzte Applikation prädestiniert. Dazu zählen u.a. spinale Fusionen bei degenerativen Wirbelsäulenerkrankungen, die Regeneration von Schädelknochen, kiefer- und gesichtschirurgische Eingriffe, sowie die Behandlung komplizierter Knochenfrakturen der Extremitäten. Für diese Anwendungen konnte der positive Effekt von rhBMP-2 (rh - rekombinant human) auf die Knochenneubildung und damit auf den Heilungsprozess bereits eindrucksvoll in mehreren Tiermodellen demonstriert werden [Lane et al., Clin. Orthop. 361 (1999), 216-227; Marden et al., J. Biomed. Mat. Res. 28 (1994), 1127-1138; Mayer et al., Plast. Recontr. Surg. 98 (1996), 247-259; Sandhu und Boden, Orthop. Clin. North Am. 29 (1998), 621-631; Toriumi et al., Arch. Otolyryngol. Head Neck Surg. 117 (1991), 1101-1112; Yasko et al., J. Bone Joint Surg. (Am.) 74 (1992), 659-670]. Für spinale Fusionsoperationen liegen sogar schon die ersten klinischen Studien vor [Boyne et al., Int. J. Periodontics Restorative Dent. 17 (1997), 11-25; Howell et al., Int. J. Periodontics Restorative Dent 17 (1997), 124-139]. Ebenfalls sinnvoll ist der Einsatz von rhBMP-2 für die Fixierung von künstlichen Gelenken oder Verschraubungen im umgebenden Knochengewebe, wie sie beispielsweise zur Stabilisierung von Frakturen verwendet werden.

Dem routinemäßigen Einsatz von BMPs in der klinischen Praxis stehen neben den noch fehlenden Studien - und auch diesen selbst - vor allem die hohen Kosten für die Proteingewinnung im Weg. Durch das in der vorliegenden Erfindung beschriebene Verfahren lässt sich biologisch aktives BMP-2, wie auch verwandte BMPs, mit geringem technischen Aufwand und bisher nicht für möglich gehaltenen Ausbeuten kostengünstig in hoher Reinheit rekombinant herstellen.

Humanes BMP-2 wird im Körper in Form eines Präproproteins von 396 Aminosäuren Länge synthetisiert. Das Signalpeptid (Präsequenz) dient *in vivo* zum Transport der nascierenden Polypeptidkette ins endoplasmatische Retikulum (ER). Nach dem Import in das ER faltet das Protein zu seiner nativen Konformation, es werden die Disulfidbrücken ausgebildet (Cys$^{296}$-Cys$^{361}$, Cys$^{325}$-Cys$^{393}$, Cys$^{329}$-Cys$^{395}$, intermolekular: Cys$^{311}$-Cys$^{360}$), und es finden posttranslationale Modifikationen statt. Zu letzteren gehören die Glycosylierung von Asparaginresten und die zum Teil unvollständige Abspaltung des Propeptids [Israel et al., Growth Factors 7 (1992), 139--150]. Die C-terminalen 114 Aminosäuren (Gln$^{283}$-Arg$^{396}$) des Vorläuferproteins bilden das reife BMP-2, dessen biologisch aktive Form ein disulfidverbrücktes (Cys$^{360}$-Cys$^{360}$) Homodimer ist.

Die Gewinnung von BMP-2 aus Knochengewebe ist mit erheblichem Aufwand und nur geringen Ausbeuten verbunden (40 μg BMP-Gemisch aus 40 kg bovinen Knochenpulvers) [Wozney et al., Science 242 (1988), 1528-1534]. Zudem ist die Isolierung aus humanem Knochenmaterial ethisch fragwürdig und birgt Gefahren durch Verunreinigung mit Pathogenen (Prionen, HCV etc.). Die Verwendung homologer Proteine aus Schweinen oder Rindern ist aus immunologischer Sicht ebenfalls riskant.

Daher wird die rekombinante Herstellung von BMP-2 favorisiert. Dafür kommen vorrangig zwei Möglichkeiten in Betracht. Zum einen kann das Protein in eukaryotischen Expressionssystemen gewonnen werden [Wang et al., Proc. Natl. Acad. Sci. USA 87 (1990), 2220-2224]. Nachteile sind hierbei die relativ geringen Mengen rekombinanten Proteins (25 μg partiell gereinigtes BMP-2 je 1 l Medium [Wozney et al., Science 242 (1988), 1528-1534]), der verhältnismäßig hohe technische Aufwand und die daraus resultierenden Kosten.

Der zweite Weg beinhaltet die Verwendung prokaryotischer Expressionssysteme. Deren Vorteile liegen in der einfachen technischen Handhabung, den geringen Kosten und der Gewinnung sehr hoher Proteinmengen. Im Gegensatz zu Eukaryoten sind Bakterien jedoch nicht in der Lage, das Vorläuferprotein korrekt zu prozessieren. Daher beschränkt sich das bislang verwendete Verfahren auf die Expression der reifen Domäne von BMP-2 ($Gln^{283}$-$Arg^{396}$). Diese fällt in den Bakterien in einer unlöslichen, biologisch inaktiven Form an, da u.a. wegen der reduzierenden Bedingungen des Cytosols, die Disulfidbrücken des Proteins nicht ausgebildet werden können. Diese unlöslichen Proteinaggregate werden als *inclusion bodies* (IBs) bezeichnet, deren Isolierung und Reinigung beispielsweise von Marston [Biochem. J. 240 (1986), 1-12] beschrieben worden ist.

Das Verfahren von Ruppert et al., [Eur. J. Biochem. 237 (1996), 295-302] zur Naturierung von reifem BMP-2 basiert auf dem US-Patent 5,650,494 [Cerletti et al., 1997], welches sich auf TGF-β-ähnliche Proteine bezieht, wozu auch BMP-2 gehört. Das Verfahren beinhaltet die Naturierung der monomeren, denaturierten reifen Form TGF-β-ähnlicher Proteine in Gegenwart eines milden Detergens zur biologisch aktiven Konformation. Im Fall von BMP-2 wurden so nur Ausbeuten von 0,2 mg aktiven Proteins je 1 g Zellen erhalten [Ruppert et al., Eur. J. Biochem. 237 (1996), 295-302].

Die vorliegende Erfindung betrifft die Verwendung von Pro-BMP-2 gemäß Tabelle 2 mit der kompletten Pro-Sequenz, die an das reife BMP-2 gebunden ist, zur Herstellung eines Arzneimittels zur Förderung der Knochenheilung und zur Herstellung von Konochenersatzmaterial oder Implantaten. Andere Aspekte der Erfindung betreffen ein Arzneimittel zur Förderung des Knochenwachstums, gekennzeichnet durch einen Gehalt an Pro-BMP-2 gemäß Tabelle 2 mit der vollständigen Pro-Sequenz $Gly^{22}$-$Arg^{284}$ als Wirkstoff und ein Verfahren zur Herstellung eines Arzneimittels zur Förderung des Knochenwachstums, dadurch gekennzeichnet, dass Pro-BMP-2 gemäß Tabelle 2 mit der vollständigen Pro-Sequenz $Gly^{22}$-$Arg^{284}$ in eine zur Verabreichung geeignete Zubereitung formuliert wird.

Es wird ein Verfahren beschrieben, mit welchem es möglich ist, eine über 25fach höhere Ausbeute an aktivem Protein, bezogen auf die induzierte Zellmasse, zu erzielen. Verglichen mit der Expression in eukaryotische Zellen ist die Ausbeute sogar um etwa drei Größenordnungen höher.

Die Ausbeuten bei der Naturierung von in *E. coli* recombinant exprimierten reifen BMPs sind aufgrund des komplizierten Disulfidmusters (Cystinknoten) sehr gering. Grundlage des beschriebenen Verfahrens ist die Hypothese, dass das BMP-2-Propeptid, wie auch die Propeptide homologer Proteine, für die korrekte Faltung der reifen Domäne mitverantwortlich ist.

Das beschriebene Verfahren zur rekombinanten Herstellung eines biologisch aktiven Proteins der TGF-β-Superfamilie, ist dadurch gekennzeichnet, dass (a) ein Protein, dessen Aminoterminus aus der Prosequenz eines Proteins der TGF-β-Superfamilie oder Teilen davon besteht, an den sich die reife Domäne dieses oder eines anderen Proteins der TGF-β-Superfamilie, das mindestens 35 % Homologie zu reifem BMP-2 aufweist, anschließt, in Prokaryonten unter Bedingungen exprimiert wird, bei denen wenigstens ein Teil des Proteins in Form von Einschlusskörpern erhalten wird, (b) die Einschlusskörper isoliert und unter denaturierenden Bedingungen solubilisiert werden, (c) das aus den Einschlusskörpern solubilisierte, denaturierte, monomere und biologisch inaktive Protein naturiert wird, wodurch die Faltung und Dimerisierung zur löslichen, biologisch aktiven Konformation ermöglicht wird und (d) gegebenenfalls nach der Naturierung die proteolytische Freisetzung des reifen Proteins aus seiner Proform erfolgt.

Unsere in vitro-Renaturierungsversuche haben gezeigt, dass es erfindungsgemäß möglich ist, auch in Abwesenheit von Detergentien bei Verwendung der hypothetischen Proform von BMP-2 ($Gly^{20}$-$Arg^{396}$) und anschließender Abspaltung des Propeptids ($Gly^{20}$-$Arg^{282}$), mit geringem Aufwand große Mengen an biologisch aktivem drBMP-2 zu erhalten. Unter drBMP-2 ist hierbei die C-terminale Domäne von BMP-2 von $Lys^{290}$, $Arg^{291}$ bzw. $Leu^{292}$ (siehe **Beispiel 2.2.1**) bis $Arg^{396}$ zu verstehen, deren biologische Aktivität von Koenig et al., [Mol. Cell Biol. 14 (1994), 5961--5974] beschrieben wurde. Die Aminosäuren ($Gln^{283}$ bis $Arg^{289}$, $Lys^{290}$ bzw. $Arg^{291}$) vermitteln die Bindung von BMP-2 an Heparin.

Diese eher unspezifische Wechselwirkung moduliert zwar die Wirkung von BMP-2, ist jedoch für dessen biologische Aktivität nicht essentiell [Ruppert et al., Eur. J. Biochem. 237 (1996), 295-302].

Erfindungsgemäß kann als Prosequenz bzw. Propeptid die gesamte Prosequenz ($Gly^{22}$-$Arg^{284}$) gemäß Tabelle 2 verwendet werden.

Um die Expressionsrate von *probmp-2* in Prokaryoten zu erhöhen ist es erfindungsgemäß möglich, am 5'-Ende der dieses Gen codierenden Sequenz eine tag-Sequenz, z.B. einen Histidin-Tag, anzufügen, oder die Codons von prob*mp-2* durch Mutagenese für die prokarytische Expression zu optimieren. Auch die Expressionsverstärkung gemäß US-Patent 5,336,602 [Brinkmann et al., 1993] sowie eine Kombination aus diesen Methoden ist möglich.

Bei der Überexpression von rekombinantem pro*bmp*-2 im Cytoplasma von Prokaryoten fällt das Protein dort in Form inaktiver, unlöslicher Aggregate (*inclusion bodies*-IBs) an. Zu ihrer Isolierung werden die Zellen nach der Fermentation zunächst durch eines oder mehrere der üblichen Verfahren (z.B.: Hochdruckdispersion, enzymatische Lyse oder Ultraschall) aufgeschlossen, was vorzugsweise in einer neutralen bis schwach sauren Pufferlösung geschieht, beispielsweise 0,1 M Tris/HCl pH 7,0.

Die zelluläre DNA wird durch mechanische, chemische oder, vorzugsweise, enzymatische Behandlung in nicht mit den IBs cosedimentierende Fragmente zerlegt. Unlösliche Zellbestandteile, einschließlich der IBs, werden dann in beliebiger Weise von den löslichen abgetrennt, wobei die Separation durch Zentrifugation favorisiert wird. Das Pellet wird mit Lösungen gewaschen, die aufgrund ihrer Zusammensetzung bzw. ihrer Zusätze, wie z.B. Detergentien, in der Lage sind, störende Zellproteine und Membranbausteine jedoch nicht das rekombinante proBMP-2 aus den IBs zu solubilisieren. Der verbleibende unlösliche, proBMP-2-haltige Anteil wird nachfolgend solubilisiert, naturiert und enzymatisch prozessiert.

Vor der Naturierung werden die IBs solubilisiert, wobei gebräuchliche Denaturierungsmittel (chaotrope Substanzen) bzw. Kombinationen aus diesen zum Einsatz kommen. Dazu zählen u.a. Guandiniumsalze, z.B. GdmCl oder GdmSCN, sowie Harnstoff und dessen Derivate. Die Konzentration des Denaturierungsmittels bzw. -gemisches ist dabei so einzustellen, dass das rekombinante, schwer lösliche Protein vollständig solubilisiert werden kann. Im Fall von Guanidiniumchlorid liegen diese Konzentrationen im Bereich von 3-8 M und in dem von Harnstoff bei 6-10 M. Um ferner das so denaturiert lösliche, cystinhaltige Protein vollständig zu monomerisieren ist der Zusatz eines Reduktionsmittels, wie z.B. Dithiothreitol (DTT) oder β-Mercaptoethanol, oder einer Kombination aus Reduktionsmitteln bevorzugt, wobei der pH des Solubilisierungspuffers vorzugsweise zwischen 8 und 9 eingestellt wird. Im Anschluss an die Solubilisierung oder parallel zu dieser ist es außerdem möglich, die freien Cysteine, beispielsweise mit oxidiertem Glutathion, kovalent zu modifizieren. Nach der Solubilisierung bzw. der optionalen kovalenten Modifikation der Cysteine werden der pH des IB-Solubilisats vorzugsweise auf 5 oder weniger eingestellt und, wiederum optional, überschüssige Redoxäquivalente durch Dialyse entfernt, wobei der Dialysepuffer, abgesehen vom Reduktionsmittel, zweckmäßigerweise die gleiche Zusammensetzung hat wie der Solubilisierungspuffer. Schließlich werden die während des Solubilisierungsprozesses unlöslich verbliebenen Bestandteile abgetrennt (Zentrifugation oder Filtration) und verworfen. Der optionale Modifizierungsschritt und die Entfernung des überschüssigen Reduktionsmittels ermöglichen ggf. eine Vorreinigung des IB-Solubilisats über Immobilisierte Metall- Affinitätschromatographie (IMAC), sofern die Expression von proBMP-2 mit N-terminalem Histidin-Tag erfolgte.

Der beschriebene Prozess der Naturierung von rh-proBMP-2 beinhaltet die Verringerung der Konzentration der(/des) Denaturierungsmittel(s) auf ein nicht oder nur schwach denaturierend wirkendes Niveau (z.B. ≤ 0,5 M GdmCl). Das geschieht bevorzugt durch langsam kontinuierliche oder schrittweise Verdünnung des Solubilisats in Naturierungspuffer. Um die Ausbeute an biologisch aktivem Protein zu maximieren ist darauf zu achten, dass die Durchmischung dabei möglichst rasch und gründlich erfolgt, was z.B. durch starkes Rühren gewährleistet ist.

Eine Alternative zur Verdünnung ist die Dialyse des Solubilisats gegen Naturierungspuffer. Die Bedingungen sind in jedem Fall so zu wählen, dass die Bildung von Proteinaggregaten minimal ist. Bereits während der initialen Verdünnung des Denaturierungsmittels kann eine Naturierung des Proteins stattfinden. Die Endkonzentration von rh-proBMP-2 im Naturierungspuffer beträgt nach Verdünnung bzw. Dialyse 10-1000 μg/ml, vorzugsweise 50-250 μg/ml.

Eine optimale Proteinkonzentration ist dann gewährleistet, wenn die unproduktive Aggregation falsch gefalteter Polypeptidketten und Faltungsintermediate (ein Prozess ≥ 2. Ordnung [Kiefhaber et al., Biotechnology (N.Y.) 9 (1991), 825-829]) deutlich langsamer abläuft als die Faltung der einzelnen Polypeptidketten zur nativen Konformation (Prozess 1. Ordnung).

Der pH des verwendeten Naturierungspuffers beträgt 6,5-10, idealerweise 8-9. Als Puffer kommen somit alle, einen neutralen bis alkalischen $pK_a$ aufweisenden, vorzugsweise Tris- und Phosphatpuffer, sowie Kombinationen aus diesen in Frage. Ferner ist der Zusatz niedermolekularer Hilfsstoffe, welche die Naturierungsausbeute erhöhen [US-Patent 5,593,865, Rudolph et al., 1995], zum Naturierungspuffer zweckmäßig. Besonders geeignet ist die Naturierung in Gegenwart von L-Arginin, wobei Konzentrationen von 0,2-2,0 M , bevorzugt 0,5-1,5 M L-Arginin, eingesetzt werden. Eine weitere Komponente des Naturierungspuffers ist erfindungsgemäß die Kombination von reduzierter und oxidierter Form einer oder mehrerer Thiolverbindungen. Solche Kombinationen sind beispielsweise reduziertes (GSH) und oxidiertes (GSSG) Glutathion, Cystein und Cystin, Cysteamin und Cystamin, sowie β-Mercaptoethanol und 2,2'-Hydroxyethyldisulfid. Mit Hilfe eines solchen künstlichen Redoxsystems lässt sich die Naturierung des Proteins thermodynamisch kontrollieren. Die für die korrekte Faltung der Polypeptidketten notwendige Möglichkeit zur Ausbildung der Disulfidbrücken [Creighton, Biochemistry 378 (1997), 731-744] wird durch das Vorhandensein der oxidierten Form, die Möglichkeit zum Wiederaufbrechen falsch gebildeter Cystine *(reshuffling)* durch das der reduzierten Form der Thiolkomponente gewährleistet [Creighton et al., Trends Biotechnol. 13 (1995), 18-23].

Bevorzugt wird Glutathion in reduzierter (GSH) und oxidierter (GSSG) Form in Konzentrationen von jeweils bis zu 10 mM verwendet, idealerweise in einem Verhältnis von 5 mM GSSG zu 2 mM GSH.

Der Naturierungspuffer enthält bevorzugt einen oder mehrere Komplexbildner, wie EDTA (Ethylendiamintetraessigsäure), in Konzentrationen von bis zu 20 mM (bevorzugt ca. 5 mM), um zum einen die unkontrollierte, beschleunigte Oxidation der reduzierten Thiolkomponente durch Metallionen zu unterbinden und zum anderen Metalloproteasen, durch die das Renaturat kontaminiert sein kann, zu inhibieren.

Die Naturierung erfolgt zweckmäßig bei Temperaturen von 0-20 °C über 1-21 d, vorzugsweise 5-10 °C, 5-10 d.

Die relativ langen Renaturierungszeiten lassen sich durch die kovalente Dimerisierung der proBMP-2-Polypeptidketten erklären. Unsere Versuche haben gezeigt, dass für diesen Vorgang unter den Bedingungen der vorliegenden Erfindung nicht die Proteinkonzentration, sondern die Oxidation von freien Thiolgruppen geschwindigkeitsbestimmend ist.

Nach Abschluss des Naturierungsprozesses wird erfindungsgemäß gegen einen vorzugsweise sauren (pH ≤ 5) Puffer dialysiert, der keine niedermolekularen Hilfsstoffe im Sinne der Beschreibung enthält. Der Dialysepuffer entspricht dabei dem beschriebenen Verfahren, wenn durch die Dialyse falsch gefaltete Polypeptidketten und Faltungsintermediate weitestgehend aggregieren und somit durch Zentrifugation, Filtration oder eine andere übliche Methode abgetrennt werden können. Vor der Dialyse ist eine Konzentrierung des Naturierungsansatzes, beispielsweise durch Cross-Flow-Filtration, zweckmäßig, an die sich eine zusätzliche Inkubationszeit unter Naturierungsbedingungen anschließen kann. Verkürzend auf die Naturierungszeit kann sich die Belüftung des Puffers auswirken, die u.U. auch beim Prozess der Konzentrierung geschieht, und die Oxidation freier Thiolgruppen beschleunigt. Dies kann ebenso durch Zusatz oxidierend wirkender Substanzen erreicht werden.

Die Trennung von monomeren, falsch gefalteten bzw. nicht dimerisierten, korrekt gefalteten Spezies von der nativen, dimeren Form des renaturierten rh-proBMP-2 kann erfindungsgemäß mit Hilfe chromatographischer Trennverfahren erfolgen, wobei vorzugsweise reversed-phase-HPLC verwendet wird. Als Säulenmaterialien eignen sich hierfür vor allem silicabasierte C4-Matrizes bzw. polymerbasierte Partikel vergleichbarer oder stärkerer Hydrophobizität, wobei ein Porendurchmesser von ≥ 300 Å aufgrund der Größe des Proteins empfehlenswert ist. Als Eluenten eignen sich die in der RP-HPLC üblichen, wie Acetonitril, 2-Propanol oder Ethanol. Es kommen dabei Ionenpaarbildner, vorzugsweise Trifluoressigsäure bzw. Perfluorbuttersäure in den gängigen Konzentrationen, zum Einsatz.

Eine weitere Möglichkeit zur Reinigung von dimeren, naiv gefalteten proBMP-2 ist der Einsatz der Heparin-Affinitätschromatographie. Dabei sind zur Erhöhung der Löslichkeit des Proteins und zur Vermeidung unspezifischer Wechselwirkungen zwischen proBMP-2 und Säulenmaterial Harnstoffkonzentrationen von 2 bis 8 mol l$^{-1}$, vorzugsweise 5 mol l$^{-1}$ als Pufferzusatz zu verwenden. HiTrap Heparin-Sepharose (Amersham Phamracia Biotech) ist dabei als Säulenmaterial besonders geeignet. Die monomeren Formen von proBMP-2 werden dabei unter 300 mol l$^{-1}$ NaCl und die dimere Form bei höheren Salzkonzentrationen von der Matrix eluiert.

Für den nicht zur TGF-β-Familie zählenden Nervenwachstumsfaktor β-NGF wurde bereits ein Verfahren zur Gewinnung des biologisch aktiven Proteins aus seiner inaktiven, denaturierten Proform in WO 00/22119 [Rattenholl et al., 2000] beschrieben. β-NGF und BMP-2 unterscheiden sich insbesondere in der Länge ihrer Prosequenz (β-NGF-103 AS; BMP-2-263 AS). Auch in der Tertiärstruktur gibt es wesentliche Unterschiede [McDonald und Hendrickson, Cell 73 (1993), 421-424]. Es ist daher nicht überraschend, dass die vorliegende Erfindung in wesentlichen Punkten von WO 00/22119 differiert.

Die für maximale Ausbeuten an korrekt gefalteten rh-proBMP-2 erforderlichen Naturierungszeiten sind wesentlich länger (1-21 d) als die für rh-proNGF (3 h). Die Proform von BMP-2 wird, wie auch die von β-NGF, *in vivo* durch Prohormonkonvertasen des Furintyps prozessiert. Diese erkennen ein dibasisches Sequenzmotiv am Ende der Prosequenz. Deren letzte Aminosäure ist Arg$^{282}$. *In vitro* kann das Propeptid von BMP-2 bzw. β-NGF durch Proteasen mit vergleichbarer Substratspezifität abgespalten werden.

Die Prozessierung des naturierten rh-proBMP-2 erfordert jedoch gänzlich andere Bedingungen als die für die Spaltung von proNGF angewandten. Obwohl die verwendete Prosequenz von BMP-2 mit 263 AS wesentlich länger als das reife Protein (114 AS) ist, hat sich bei unseren Versuchen überraschenderweise gezeigt, dass die Löslichkeitseigenschaften von proBMP-2 von dessen reifer Domäne determiniert werden proBMP-2 ist daher, im Gegensatz zum BMP-2-Propeptid allein (Gly$^{20}$-Arg$^{282}$), ohne lösungsvermittelnde Zusätze nur bei pH ≤ 5 in hohen Konzentrationen löslich. Bei saurem pH ist eine effektive Prozessierung der Proform mit Hilfe von Proteasen (Trypsin, PACE *(paired basic amino acidconverting enzymes;* nach dibasischen Motiven schneidende, eukaryotische Endopeptidasen)) nicht möglich. Um eine proteolytische Spaltung dennoch zu ermöglichen, kommen alle nicht inhibierenden Lösungsvermittler besonders Harnstoff oder Trishydroxymethylaminomethan (Tris) in Konzentrationen von 1-5 M bzw. 0,1-2 M einzeln oder in Kombination zum Einsatz, vorzugsweise 2-4 M Harnstoff in Kombination mit 0,1-1 M Tris. Mit Hilfe dieser Zusätze ist es möglich, proBMP-2 in Konzentrationen von ≥ 1 mg/ml auch im pH-Optimum (7-8) der verwendeten Proteasen in Lösung zu halten.

Die Einstellung der Pufferbedingungen für die Proteolyse erfolgt zweckmäßigerweise durch Dialyse. Erfindungsgemäß werden trypsinähnliche Proteasen favorisiert, die in einem Massenverhältnis von 1:10 bis 1:1000 (Trypsin, rh-proBMP-2) zum Einsatz kommen, bevorzugt von 1:20 bis 1:100. Inkubiert wird über einen Zeitraum von 1 min bis 24 h, bevorzugt 30 min bis 6 h, bei einer Temperatur von 0-37 °C, bevorzugt 10-25 °C. Als Puffersubstanz fungiert im Fall von Tris der Lösungsvermittler selbst und im Fall von Harnstoff zusätzlich Trispuffer in den angegebenen Konzentrationen. Eine hohe Konzentration von Tris, z.B. 0,5 M, verhindert zudem die kovalente Modifikation von Aminosäureseitenketten durch eventuell aus dem Harnstoff gebildetem Isocyanat.

Der pH wird auf einen für die betreffende Protease günstigen Wert, vorzugsweise 7-8, eingestellt. Um unspezifischen proteolytischen Abbau durch Metalloproteasen zu unterdrücken ist der Zusatz von Komplexbildnern, z.B. 1-20 mM EDTA, zweckmäßig. Die Proteolyse wird durch Zusatz eines oder mehrerer Proteaseinhibitoren, bevorzugt 1-5 mM Phenylmethylsulfonylfluorid (PMSF) oder Trypsininhibitor aus Rinderpankreas oder Sojabohnen (BPTI/SBTI) in 2-20fachem molaren Überschuss zu Trypsin, bzw. durch Einstellung von pH ≤ 5 mittels Säure abgestoppt [Rudolph *et al.,*: Folding proteins, in Protein Function: A Practical Approach, T.E. Creighton (Hrsg.), 2. Aufl. (1997), 57-99].

Der N-Terminus des reifen BMP-2 (Gln[283]-Arg[291]) ist nicht strukturiert und daher proteaseempfindlich. Er enthält zudem einige Arginin- und Lysinreste (Arg[289,291], LyS[285,287,290]). Die limitierte Proteolyse mit Trypsin, das bevorzugt nach diesen basischen Aminosäureresten schneidet, führt daher auch zur Abspaltung des flexiblen N-Terminus von reifem BMP-2. Die biologische Aktivität des erhaltenen drBMP-2 wurde im Tierversuch nachgewiesen. Es ist möglich, ggf. durch *Protein Engineering* eine zusätzliche Disulfidbrücke zur Fixierung und Stabilisierung des N-Terminus von reifem BMP-2 einzuführen, z.B. ähnlich wie sie im verwandten TGF-β vorkommt, um so die Heparinbindestelle von BMP-2 vor proteolytischem Abbau zu schützen. Unsere Versuche zur limitierten Proteolyse von rh-proBMP-2 in Abwesenheit der beschriebenen Lösungsvermittlerkonzentrationen bei dann entsprechend geringen Proteinkonzentrationen haben gezeigt, dass das Propeptid aus mindestens einer, nach Abspaltung vom reifen Protein noch immer weitgehend proteaseresistenten Domäne besteht. In den verwendeten Konzentrationen der zum Einsatz kommenden Lösungsvermittler wirken diese selbst z.T. schwach denaturierend. Das hat zur Folge, dass das nicht durch intrachenare Disulfidbrücken stabilisierte Propeptid von BMP-2 strukturell destabilisiert, proteaselabil und somit unter den beschriebenen Bedingungen der limitierten Proteolyse abgebaut werden kann.

Für die Reinigung der Proteolyseprodukte bietet sich aufgrund der extremen Hydrophobizität von nativem BMP-2 [Scheufler et al., J. Mol. Biol. 287 (1999), 103-115] die Reinigung über hydrophobe Wechselwirkungen an. Es kommen hierfür bevorzugt Säulenmaterialien zum Einsatz, an die das erhaltene drBMP-2 stark bindet, wohingegen die übrigen Abbauprodukte, sowie die verwendete Protease bzw. der Inhibitor mit dem Material nur schwach wechselwirken. Entsprechend ist es möglich, die Pufferbedingungen nach der Proteolyse so zu verändern, dass das drBMP-2 präzipitiert und die übrigen Abbauprodukte, die Protease bzw. ihr(e) Inhibitor(en) in Lösung verbleiben. Das präzipitierte drBMP-2 kann dann, nach Separation vom Überstand, in geeigneten Puffern bzw. Lösungsmitteln wieder solubilisiert werden. Bevorzugt erfolgt diese Veränderung zu präzipitierenden Bedingungen in Gegenwart von geeignetem Säulenmaterial, besonders "Fractogel EMD Phenyl S" (Merck) oder vergleichbaren Materialien, wie Phenylsepharose (Pharmacia), durch Dialyse. Wird das Material in großem Überschuss eingesetzt, kann erreicht werden, dass das Protein an dieses bindet bevor es aggregiert. Nachdem das Säulenmaterial auf diese Weise mit Protein beladen worden ist, können die unerwünschten Komponenten durch geeignete Waschschritte entfernt werden. Die Elution des drBMP-2 erfolgt zweckmäßigerweise unter mild bis stark denaturierenden, aber keinesfalls reduzierenden Bedingungen. Als Elutionsmittel eignen sich je nach Säulenmaterial dementsprechend u.a. Arginin, Harnstoff oder Guanidiniumsalze, die in niedrigen Konzentrationen auch zum Waschen des Säulenmaterials Verwendung finden können. Nach der Elution werden die drBMP-2 enthaltenen Fraktionen ggf. vereinigt und besonders bevorzugt gegen 0,1 % Trifluoressigsäure (TFA) oder Ammoniumacetat dialysiert, welche für die schließliche Applikation von BMP-2 sehr geeignete Lösungsmittel darstellen. Es ist auch möglich, die Proteolyseprodukte über *reversed* phase-Materialien, bevorzugt C$_4$-RP-HPLC, zu trennen, wobei die Elution vorzugsweise mit 0,1 % TFA im Acetonitrilgradienten erfolgt.

Renaturiertes und nach Abspaltung der pro-Sequenz erhaltenes reifes BMP-2 kann in der dafür üblichen Weise zur Förderung des Knochenwachstums verwendet werden. Überraschenderweise ist jedoch auch das renaturierte proBMP-2 selbst, welches die gesamte oder eine verkürzte pro-Sequenz noch enthält, in seiner biologischen Wirksamkeit dem reifen BMP-2 äquivalent und kann wie dieses eingesetzt werden. Ein Gegenstand der Erfindung ist daher auch ein proBMP-2 als Wirkstoff enthaltendes Arzneimittel zur Förderung des Knochenwachstums. Es kann die komplette oder eine trunkierte pro-Sequenz enthalten. Bevorzugt wird erfindungsgemäß eine Kombination von proBMP-2 mit verschiedenen medizinischen Implantatmaterialien für den vorübergehenden Knochenersatz (Knochenersatzmaterialien) sowie für den permanenten Knochen- und Gelenkersatz (Metallimplantate, wie Hüft- und Kniegelenksendoprothesen) in Traumatologie, Orthopädie, HNO, MKG und Neurochirurgie verwendet. Hierbei kann zum einen proBMP-2 als nachträgliche Implantatbeschichtung in der Art eines dünnen auf allen inneren und äußeren Oberflächen anhaftenden Proteinfilms mit und ohne Haftvermittler (z.B. Silan) zur Anwendung kommen. Zum zweiten kann proBMP-2 als integraler Bestandteil von Implantaten verwendet werden, indem dieses den nicht festen Edukten solcher durch chemische Polymerisation oder Aushärtung herstellbaren festen Endprodukten zugesetzt wird (z.B. Calciumphosphatzemente wie Norian SRS, Ostim u.a.).

Als Implantate für den transienten Knochenersatz kommen anorganische und organische Materialien natürlichen und künstlichen Ursprungs sowie Mischformen aus beiden in Frage. Beispiele für anorganische Knochenersatzmaterialien stellen Keramiken aus Hydroxylapatit, beta-Tricalciumphosphat, alpha-Tricalciumphosphat, Glaskeramiken, Biogläser, Calciumcarbonate, Calciumsulfate und sonstige nicht aufgeführte Calciumsalze in kristalliner und amorpher Form einschließlich injizierbarer Calciumphosphat-basierter Knochenzemente dar. Geeignete organische Materialien stellen unter anderem Kollagene, Knochengewebe, demineralisierte Knochenmatrix, gelierfähige Polysaccharide wie Hyaluronsäure, biokompatible Kunststoffe, wie Polymilchsäure, Polymethylacrylate, Polyethylenglykole u.a.m. in verschiedensten Misch- und Verarbeitungsformen dar. Für den dauerhaften Knochenersatz kommen Endoprothesen und andere Metallimplantate wie solche für die Wirbelkörperstabilisierung in Betracht. Üblicherweise handelt es sich hierbei um Produkte aus verschiedenen Edelstählen und Titanlegierungen, die zum Teil auch mit Hydroxylapaptibeschichtungen versehen sind.

Weitere Gegenstände der Erfindung sind daher ein ProBMP-2 gemäß Tabelle 2 mit der vollständigen Pro-Sequenz $Gly^{22}$-$Arg^{284}$ enthaltendes Arzneimittel und ein Verfahren zur Herstellung eines Arzneimittels zur Förderung des Knochenwachstums, wie in den Patentansprüchen definiert. Die vollständige Sequenz von proBMP-2 von $Gly^{20}$-$Arg^{396}$, einschließlich 20 Aminosäuren His-Tag und dem Startcodon Met ist in Tabelle 2 dargestellt.

Die Aminosäuren Met1 bis His20 sind Bestandteil des von pET-15b codierten His-Tags. Die Prosequenz von BMP-2 beginnt bei Gly22. Zwischen His-Tag und Prosequenz befindet sich das zusätzliche Methionin. Gly22 bis Arg 398 in der hier dargestellten Sequenz entsprechen Gly20 bis Arg396 im primären Translationsprodukt der humanen Prä-ProBMP-2-cDNA, auf welches sich die Sequenzangaben im Text beziehen.

Die folgenden Beispiele erläutern die Erfindung in Verbindung mit der Zeichnung. In dieser stellen dar:

| | |
|---|---|
| Abbildung 1 | ein SDS-PAGE-Elektropherogramm |
| Abbildung 2 | UV-CD-Spektren |
| Abbildung 3 | Emissionsspektren, jeweils von proBMP-2 |
| Abbildung 4 | ein SDS-PAGE-Elektropherogramm |
| Abbildung 5 | ein RP-HPLC-Chromatogramm |
| Abbildung 6 | UV-CD-Spektren, |
| Abbildung 7 | MALDI-TOF-Massenspektrogramm, jeweils von drBMP-2 |
| Abbildung 8 | ein RP-HPLC Diagramm von rh-proBMP-2 |
| Abbildung 9 | histologischer Schnitt eines rh-proBMP-2-Implantats. Sowohl die innere als auch die äußere Oberfläche der Keramik sind mit Knochensubstanz bedeckt. Das Poreninnere enthält Knochenmark mit zahlreichen Fettzellen (Implantatmaterial - dunkel, Knochen - hellblau). Links unten sind einige Muskelfasern (dunkelblau) sowie Bindegegebe zu sehen. |
| Abbildung 10 | Histologischer Schnitt eines rh-proBMP-2-Impantats in starker Vergrößerung. Das Knochenmaterial (links oben bis rechts unten, hellblau) befindet in sehr engem Kontakt zu dem von ihm bedeckten Keramikmaterial (rechts, grau). Zwischen Knochensubstanz und hematopoietischem Gewebe befinden sich aktive Osteoblasten in einer Reihe. |
| Abbildung 11 | ein Vergleich der Aktivität der AP in den Implantaten. |

## Beispiele

### Ausführungsbeispiel 1: Herstellung von proBMP-2

Klonierung der pro*bmp*-2-cDNA in einen *Escherichia coli*-Expressionsvektor

Zur Klonierung der pro*bmp*-2-cDNA wurde das T7-Expressionssystem der Firma Novagen [Studier und Moffat, J. Mol. Biol. 189 (1986), 113-130] verwendet.

Als Ausgangs-DNA diente der pcDNA3-Vektor mit inserierter präpro*bmp*-2-cDNA (Boehringer-Mannheim GmbH). Durch Polymerasekettenreaktion (PCR) wurde die für die Aminosäuren $Gly^{20}$ bis $Arg^{396}$ von BMP-2 kodierende DNA erhalten. Dabei wurden Mutageneseprimer verwendet, mit deren Hilfe am 5'-Ende ein Methionincodon für den Translationsstart eingeführt und das Stopcodon am 3'-Ende in ein für *E. coli* typisches überführt sowie durch ein zweites Stopcodon ergänzt wurde. Außerdem enthielten die Primer eine *Nde*I- (5') bzw. eine *Bam*HI-Schnittstelle (3'-Ende). Das ermöglichte die anschließende Klonierung in den Expressionsvektor pET-15b (Novagen). Dieser Vektor codiert zusätzlich einen N-terminalen 6-Histidin-Tag, durch welchen sich die Expressionsraten in *E. coli* beträchtlich steigern ließen.

Die Nucleotidsequenz wurde nach Abschluss der Klonierung mittels DNA-Sequenzierung [Sanger et al., Proc. Natl. Acad. Sci. USA 74 (1977), 5463-5467] überprüft.

Folgende Primer wurden verwendet:

*forward primer* "PrimerFwProBMP":

```
                        NdeI
        5'-cg gaa ttc ca↓t atg ggt gcg gct ggc ctc gtt cc-3'
                        Met Gly Ala Ala Gly Leu Val
                        H₂N—→
```

*reverse primer* "PrimerRevBMP":

```
            BamHI
        5'-cc g↓ga tcc tta cta gcg aca ccc aca acc-3'
                Stp Stp Arg Cys Gly Cys Gly
                HOOC←—
```

Als Wirtsstamm diente *E. coli* BL21 (DE3). Das Chromosom dieser Bakterien enthält das Gen für die T7-RNA-Polymerase, welches unter Kontrolle des lac-Promotors steht. Die Expression der Polymerase kann somit durch Isopropyl-β-D-thiogalactosid (IPTG) induziert werden. Das pro*bmp*-2-Gen wird von einem T7-Promotor kontrolliert. Die Induktion mit IPTG führt folglich zur Bildung von proBMP-2. Zur weiteren Erhöhung der Expressionsrate wurden die Zellen mit pUBS520 cotransformiert. Dieses Plasmid enthält das Gen für eine in *E. coli* seltene Arginin-tRNA *(dnaY).* Die von dieser tRNA erkannten Argininoodons (AGA und AGG) finden sich dagegen besonders häufig in eukaryotischen Genen, so auch in pro*bmp*-2. Die Expressionsrate solcher Gene in *E. coli* hängt u.a. von der Verfügbarkeit dieser tRNA ab [Brinkmann et al., Gene 85 (1989), 109-114].

**Expression von humanem proBMP-2 in *E.coli***

Für die Anzucht des rekombinanten Bakterienstammes wurde ein geeignetes Volumen 2·YT-Medium, i.d.R. 1,5 l, mit 100 μg/ml Ampicillin und 50 μg/ml Kanamycin versetzt und mit einer Einzelkolonie beimpft.

**2-YT-Medium (1 l):**
17 g Trypton
10 g Hefeextrakt
5 g NaCl

Die Kultur wurde bei 37 °C und 160-200 rpm geschüttelt. Induziert wurde mit 1 mM IPTG bei einer $OD_{600}$ von 1-1,3. Anschließend wurde für weitere 3-4 h bei gleicher Temperatur geschüttelt und so reproduzierbar Zelldichten nach Induktion von $OD_{600}$ 3-3,5 erzielt. Die Zellernte erfolgte durch Zentrifugation bei 10.000 g. 3-3,5 g Zellen (Feuchtmasse) je 1 l Medium konnten so gewonnen werden. Alternativ zum sofortigen Zellaufschluss wurden die Zellen zunächst in flüssigem Stickstoff und danach bei -20 °C eingefroren.

**Isolierung der *inclusion bodies* (IBs)**

Bei der rekombinanten pro*bmp*-2-Expression bilden sich in den Bakterienzellen Aggregate, *inclusion bodies* (IBs). Die Präparation dieser IBs erfolgte nach Rudolph et al.,: "Folding proteins", in "Protein Function: A Practical Approach", T.E. Creighton (Hrsg.), 2. Aufl. (1997), 57-99.

Je 5 g Zellpellet wurden in 25 ml 100 mM Tris/HCl pH 7,0, 1 mM EDTA resuspendiert. Nach Hinzufügen von 1,5 mg Lysozym je 1 g Zellfeuchtmasse folgte eine dreissigminütige Inkubation der Zellsuspension bei 4 °C. Schließlich wurden die Zellen mittels Hochdruckdispersion (Gaulin-Homogenisator) aufgebrochen. Nach Zugabe von 3 mM $MgCl_2$ und 10 μg/ml DNase wurde das Homogenat für weitere 30 min bei 25 °C inkubiert. Im Anschluss daran wurden 0,5 Volumenanteile 60 mM EDTA, 6 % Triton X-100, 1,5 M NaCl pH 7,0 hinzugefügt und die Inkubation bei 4 °C für 30 min fortgesetzt, um unlösliche Zellbestandteile zu solubilisieren. Die IBs blieben unter diesen Bedingungen unlöslich und konnten durch Zentrifugation bei 39.000 g abgetrennt werden. Um überschüssiges Detergens aus den IBs wieder zu entfernen, wurden diese noch fünfmal mit 100 mM Tris/HCl pH 7,0, 20 mM EDTA gewaschen, abschließend aliquotiert und bei -20 °C gelagert.

Auf diese Weise konnten aus 1 g induzierter *E. coli*-Zellen reproduzierbar 180-210 mg IBs gewonnen werden, die bei einem Proteingehalt von 30-35 % 90-95 % rh-pro-BMP-2 enthielten.

## Solubilisierung der IBs

100-150 mg IB-Pellet wurden 4 h bei 25 °C in 1 ml 100 mM Tris/HCl pH 8,0, 6 M GdmCl, 100 mM DTT, 1 mM EDTA solubilisiert. Danach wurde der pH mit Essigsäure auf 3-4 eingestellt und die unlöslichen Bestandteile bei 16.000 g über 20 min in der Zentrifuge pelletiert. Der Überstand wurde anschließend in der Ultrazentrifuge bei 266.000 g noch einmal für 30 min zentrifugiert, um Mikroaggregate abzutrennen. Die Proteinkonzentration wurde mittels Absorptions-messung bei $\lambda$ = 280 nm bestimmt [Gill und von Hippel, Anal. Biochem. 182 (1989), 319-326] und betrug 30-52 mg/ml.

## Reinigung von proBMP-2

Wiederfindungsrate (ca. 70 %) waren dabei mit dem analytischen Maßstab vergleichbar.

Als zweite Reinigungsmethode kam Heparin-Affinitätschromatographie auf einer 1 ml HiTrap Heparin Sepharose (Amersham Pharmacia Biotech) zum Einsatz. Als Puffersystem wurden:

- 0,1 mol l$^{-1}$ Tris pH 7,56, 6 mol l$^{-1}$ Harnstoff (Puffer A) und
- 0,1 mol l$^{-1}$ Tris/HCl pH 7,5 6 mol l$^{-1}$ Harnstoff, 1 mol l$^{-1}$ NaCl (Puffer B)

verwendet. Die Säule wurde mit Puffer A über mehr als 10 Säulenvolumen äquilibriert. Verunreinigungen wurden mit einem Gradienten von 0 bis 20 % Puffer B eluiert. Die Trennung von monomeren und dimerem proBMP-2 erfolgte mit einem Gradienten von 20 bis 50 % Puffer B in 30 min bei einem Fluss von 1 ml min$^{-1}$.

## Naturierung von rh-proBMP-2 im präparativen Maßstab

Das proBMP-2-IB-Solubilisat wurde für die Naturierung mit 4 M GdmCl pH 4 verdünnt, sodass die Endkonzentration von GdmCl nach vollständiger Verdünnung des Solubilisats in Faltungspuffer 50-60 mM erreichte. Die Konzentration an proBMP-2 betrug schließlich 3-4 $\mu$M (entspricht 135-180 $\mu$g/ml).

## Faltungspuffer:

100 mM Tris/HCl pH 8,0
1 M L-Arginin
5 mM GSSG
2 mM GSH
5 mM EDTA

Nach dem Filtrieren und Entgasen des Faltungspuffers wurde das Solubilisat langsam (~ 20 ml/h) und unter Rühren hinzugegeben und der Ansatz 7 d bei 5 °C inkubiert. Die mit dem Solubilisat eingeschleppten DTT-Reste sind dabei einer Konzentration von maximal 0.4 mM GSH äquivalent.

Die Konzentrierung des Faltungsansatzes erfolgte mittels *Cross-Flow*-Filtration (Filtron Minisette, Membran: 30 kDa *open channel*). Das Konzentrat wurde anschließend gegen fünfmal 20 Volumen 50 mM Na-Acetat pH 5,0 für je 24 h dialysiert und danach aggregiertes Protein für 2 h bei 75.000 g abzentrifugiert. Die Menge des in Lösung verbliebenen Proteins wurde durch Absorption bei $\lambda$ = 280 nm bestimmt und betrug reproduzierbar ca. 50 %, bezogen auf den Proteingehalt des eingesetzten IB-Solubilisats.

Reinigung von proBMP-2

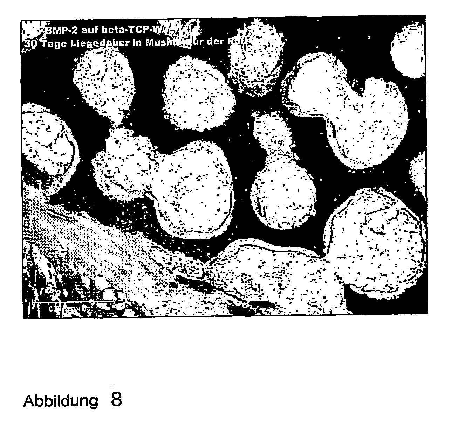

Für die analytische bzw. semipreparative Trennung der (falsch oder korrekt gefalteten) monomeren Spezies von nativem, dimerem rh-proBMP-2 wurde RP-HPLC eingesetzt. Im analytischen Maßstab wurde unter anderem eine Protein-RP-Säule (150 x 3,6 mm, YMC) mit 0,1 % Perfluorbuttersäure (HFBA) in H$_2$O bzw. ACN als Laufmittel verwendet (siehe Abbildung 8). Auch mit Trifluoressigsäure als Ionenpaarbildner wurde eine Trennung von Monomer und Dimer erreicht. Das Dimer eluiert - wie bei reifem BMP-2 - aufgrund seiner höheren Hydrophobizität später als das Monomer bzw. die monomeren, falschgefalteten Spezies. Im semipreparativen Maßstab kam eine Säule mit Source15 RPC-Material (Pharmacia) zum Einsatz. Das Trennprofil und die Wiederfindungsrate (ca. 70 %) waren dabei mit dem analytischen Maßstab vergleichbar.

**Charakterisierung von rh-proBMP-2**

**Reinheitsanalyse und Molekulargewichtsbestimmung mit SDS-Polyacrylamidgelelektrophorese**

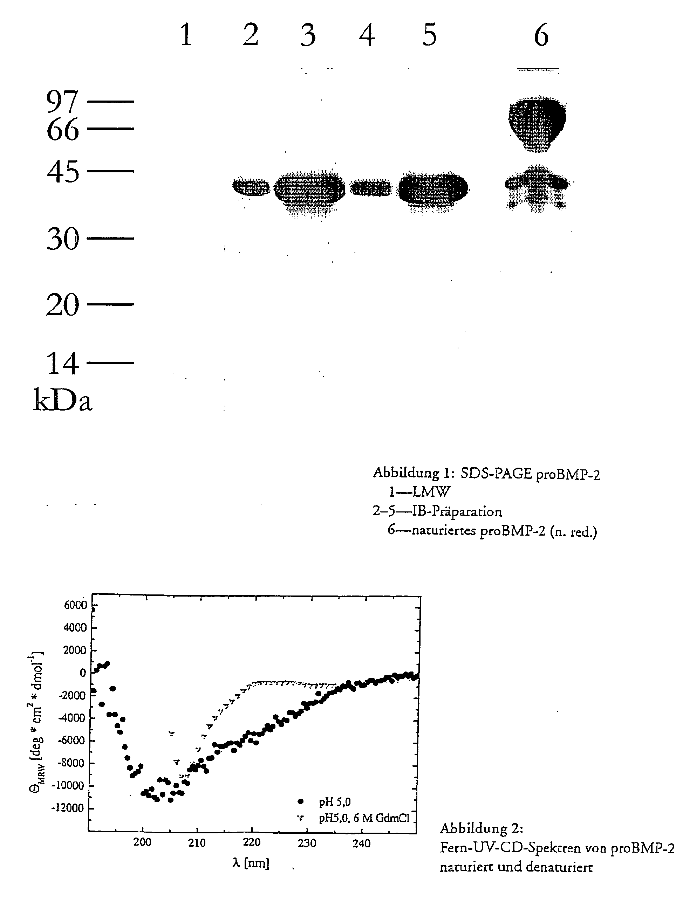

Die SDS-PAGE-Analyse ergab eine hohe Reinheit ($\geq$ 90 %; Abbildung 1, Bahn 2-5) der erfindungsgemäß präparierten proBMP-2-IBs, sodass diese ohne vorherige Reinigung für die Naturierung eingesetzt werden konnten.

SDS-PAGE unter nicht reduzierenden Bedingungen (Zugabe von Iodacetamid führt zur irreversiblen, oxidativen Modifikation freier Thiolgruppen) zeigt einen hohen kovalenten Dimerisierungsgrad ($\geq$ 90 %) von naturiertem proBMP-2 und ist ein Hinweis auf die korrekte Ausbildung der Disulfidbrücken und damit der nativen Struktur des Proteins (Abbildung 1, Bahn 6).

**N-terminaleSequenzanalyse**

Nach SDS-PAGE und Western-Blot auf eine PVDF-Memran erfolgte die N-terminale Sequenzierung [Edman und Begg, Eur. J. Biochem. 1 (1967), 80-91]. Sie ergab für naturiertes proBMP-2 folgende Aminosäuresequenz: Gly-Ser-Ser-His-His-

Das entspricht dem N-Terminus von rekombinantem proBMP-2 mit Histidin-Tag nach Abspaltung des Startmethionins.

**Circular-Dichroismus-Spektrometrie und Fluoreszenzspektroskopie**

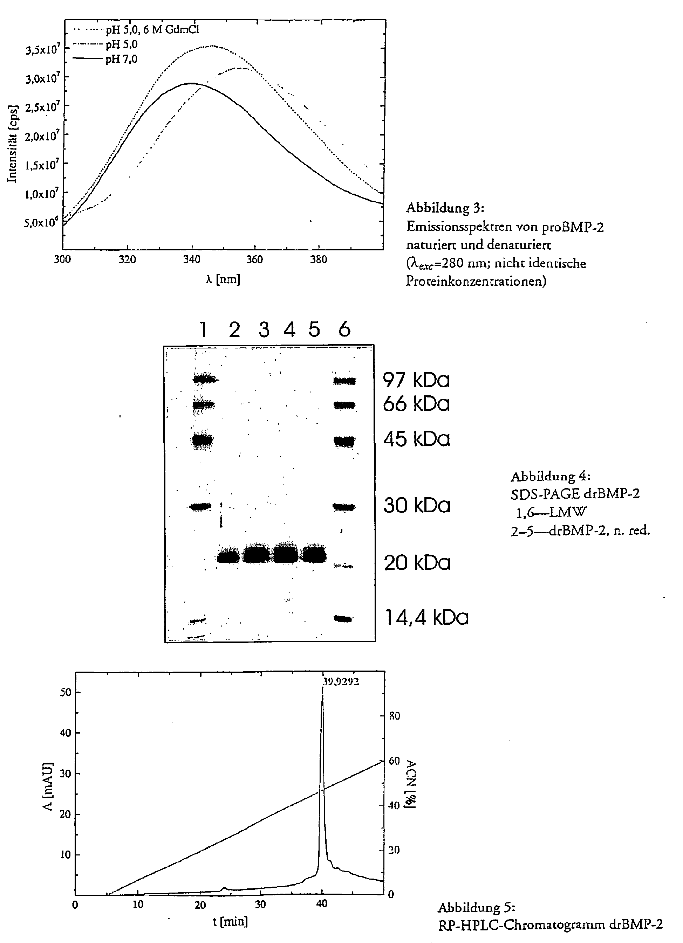

Die mit naturiertem und wieder denaturiertem proBMP-2 durchgeführten fluoreszenz- und CD-spektrometrischen Analysen lassen deutliche Hinweise auf das Vorhandensein von Sekundär- und Tertiärstruktur erkennen. Die starke Differenz zwischen der Elliptizität von naturiertem und denaturiertem proBMP-2 im Bereich um $\lambda$ = 220 nm des Fern-UV-CD-Spektrums (**Abbildung 2**) erlaubt die Feststellung, dass beim erfindungsgemäßen Prozess der Naturierung die Ausbildung von Sekundärstrukturelementen erfolgt. Die Verschiebung des Emissionsmaximums der Fluoreszenz von naturiertem proBMP-2 zu niedrigeren Wellenlängen gegenüber dem denaturierten Protein (**Abbildung 3**) ist typisch für die Ausbildung nativer Tertiärstrukturen. Dabei gelangen die Tryptophanreste des Proteins (5 Tryptophane je Polypeptidkette proBMP-2) aus der polaren Umgebung des Lösungsmittels in den apolaren Kern des sich faltenden Proteins, wo sie zudem sterisch fixiert sind. Durch den so verringerten Verlust von Anregungsenergie wird Licht höherer Frequenz emittiert. [Schmid: "Optical spectroscopy to characterize protein conformation and conformational changes." in "Protein Structure: A Practical Approach", T.E. Creighton (Hrsg.), 2. Aufl. (1996), S. 261-297]. Aus den Fluoreszenzspektren wird auch deutlich, dass die Struktur von proBMP-2 bei niedrigem pH, also im Bereich hoher Löslichkeit von reifem und proBMP-2, eine Veränderung erfährt. Wie unsere Experimente ergeben haben, kann dies auf eine partielle Entfaltung des Propeptids zurückgeführt werden (Daten nicht gezeigt).

MALDI-TOF-Massenspektrometrie und N-terminale Sequenzanalyse

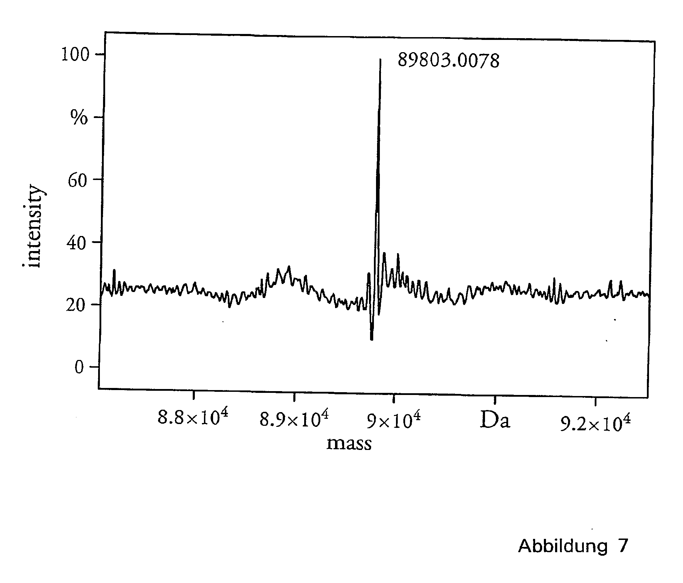

- apparente Molekularmasse: 89803,0078 Da - siehe **Abbildung 7**
- berechnete Molekularmasse (ohne Startmethionin): 89801,2 Da

**Biologische Aktivität von rh-proBMP-2 und rh-drBMP-2**

Die osteoinduktive Aktivität des modifizierten rh-proBMP-2 und rh-drBMP-2 wurde im ektopen Knocheninduktionsmodell an Laborkleintieren geprüft. Hierzu wurden definierte Mengen des jeweiligen zu prüfenden Faktors (40-200 $\mu$g; drBMP-2 bzw. proBMP-2) nach Sterilfiltration auf sterile würfelförmige, chemisch und pharmakologisch inerte keramische Implantatgrundkörper aus $\beta$-TCP aufgebracht.

**Implantatspezifikationen:**

Hersteller --- Fa. Mathys, Bettlach, Schweiz
Handelsname --- chronOs
Phasenreinheit --- > 95 % $\beta$-TCP
Porenvolumenanteil --- 72 +/- 6 %
Porengröße --- 80-650 $\mu$m
Porenstruktur --- interkonnektierend
Festigkeit --- 7,7 + /- 1,2 MPa

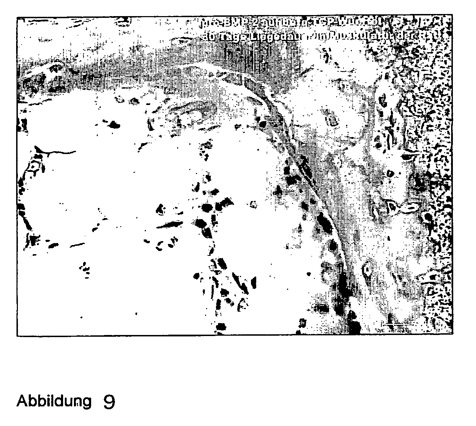

Die faktorbeschichteten Implantate sowie unbeschichtete, aber ansonsten identisch behandelten Kontrollimplantate wurden 8 ausgewachsenen Laborratten in die vordere Bauchwandmuskulatur implantiert. Hierzu wurden nach Narkotisierung mehrere, ca. 1 cm lange chirugische Schnitte in die Haut und die darunter liegende oberste Muskellage gesetzt. Durch stumpfe Präparation wurde zwischen den zwei schrägen Lagen Bauchmuskukatur ein Hohlraum erzeugt, in diesen die Implantate eingebracht und die Wunden durch chirurgische Nähte verschlossen. Nach 30 Tagen Liegedauer wurden die Tiere geopfert und die Implantate mit umgebendem Gewebe entnommen und makroskopisch und histologisch auf Knochenneubildung untersucht. Bei allen faktorbeschichteten Implantaten konnte eine Knochenneubildung, begleitet von der Bildung von hämatopoetisch aktivem Knochenmark, histologisch eindeutig festgestellt werden (siehe **Abbildungen 8 und 9**). Die biologische Wirksamkeit betrug somit für beide Faktoren jeweils 100 % (16/16 bei proBMP-2; 8/8 bei drBMP-2). Damit konnte der Nachweis der osteoinduktiven Aktivität durch die Bildung von Knochengewebe im normalerweise nicht zur Knochenbildung fähigen Muskelgewebe eindeutig erbracht werden. Qualitative Unterschiede im histologischen Ergebnis zwischen proBMP-2 und reifem drBMP-2 konnten nicht beobachtet werden. Die Kontrollimplantate führten in keinem Falle zur Knochenneubildung. Statt dessen wurden diese ausschließlich bindegewebig eingescheidet.

Ein weiterer Nachweis für die Neubildung von aktivem Knochengewebe unter dem Einfluss von rh-proBMP-2 bzw. rh-drBMP-2 wurde durch die Messung der Aktivität Alkalischer Phosphatase (AP) in den Implantaten erbracht. Hierfür wurde die Hälfte eines jeden Implantats unmittelbar nach der Explantation tiefgefroren und bei -80 °C gelagert. Für die Extraktion der Alkalischen Phosphatase und Ermittlung der enzymatischen Aktivität wurden die Implantate aufgetaut und in 0,1 M Tris/HCl, 1 % Triton X-100 pH 7,4 (bei 4 °C) mit Hilfe eines Potter S Homogenisators (B. Braun Biotech) aufgeschlossen und unlösliche Bestandteile mittels Zentrifugation abgetrennt. Der klare Überstand wurde nachfolgend für die Bestimmung der Aktivität Alkalischer Phosphatase mit dem Testkit Ecoline 25 (Merck) verwendet. Die Konzentration des bei diesem Test freigesetzten 4-Nitrophenols wurde bei 25 °C und $\lambda$ = 405 nm über 5 min verfolgt und die enzymatische Aktivität nach Gleichung 1 berechnet,

$$Akt: = \frac{Vb + Vs}{\epsilon \cdot Vs \cdot d} \cdot \frac{\Delta E}{t} \cdot 1000 \qquad (1)$$

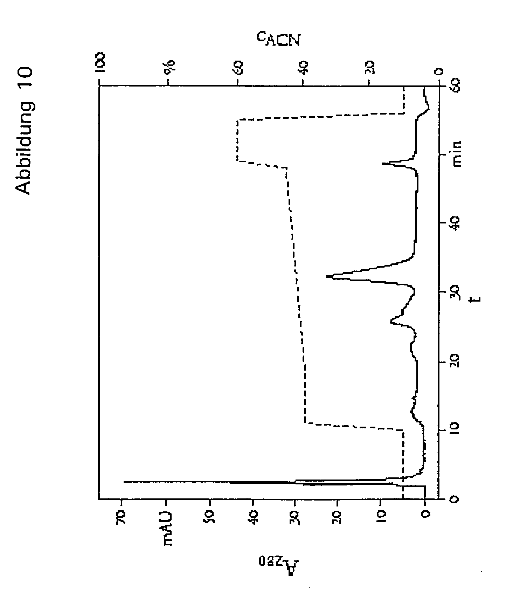

wobei Vb das Volumen des Puffers (1 ml) und Vs das der Probe (25 ml) ist. Entsprechend der Herstellerangaben wurde $\epsilon$ = 18518 l x $mol^{-1}$ x $cm^{-1}$ als molarer Absorptionskoeffizient von 4-Nitrophenol angenommen. Die Einheit der resultierenden Aktivität ist $\mu$mol x $min^{-1}$ x $ml^{-1}$. Die Ergebnisse des Tests sind in Tabelle 1 und **Abbildung 10** zusammengefasst. Die Enzymaktivität in den rh-pro/drBMP-2-haltigen Proben ist gegenüber den (-)-Kontrollen signifikant erhöht, die der rh-drBMP-2-Proben gegen denen mit rh-proBMP-2 jedoch nur geringfügig und auch nicht in jedem Fall (Ausnahmen: Tier 4, 6 und 8). Unter Berücksichtigung der unterschiedlichen Molekulargewichte von rh-proBMP-2 (ca. 45 kDa) und rh-drBMP-2 (ca. 12 kDa) sowie der Tatsache, dass die rh-proBMP-2-Proben nicht gereinigt eingesetzt wurden und somit noch falschgefaltete, monomere Spezies in einem Anteil von bis zu 10 % enthielten, lässt sich feststellen, dass rh-proBMP-2 in diesem Tiermodell eine der (trunkierten) reifen Form des Proteins vergleichbare, Knochenneubildung induzierende Aktivität besitzt.

Tabelle 1: Vergleich der Aktivität Alkalischer Phosphatase in den mit drBMP-2 oder proBMP-2 beschichteten Implantaten (in U) β-TCP

| Tier | Kontrolle | drBMP-2 | proBMP-2(a) | proBMP-2(b) |
|------|-----------|---------|-------------|-------------|
| 1 | 0,0009 | 2,1162 | 1,1654 | 1,049 |
| 2 | 0,0232 | 1,3459 | 0,7443 | 0,9126 |
| 3 | 0,017 | 2,5307 | 1,3521 | 1,4508 |
| 4 | 0,0036 | 1,0461 | 1,1934 | 1,262 |
| 5 | 0,0118 | 1,3884 | 1,0472 | 0,6895 |
| 6 | 0,0142 | 0,9195 | 1,7609 | 1,257 |
| 7 | 0,0112 | 1,776 | 1,4601 | 1,1126 |
| 8 | 0,0149 | 0,7044 | 1,6968 | 1,053 |

**Ausführungsbeispiel 2: Gewinnung von biologisch aktivem drBMP-2**

**Limitierte Proteolyse von rh-proBMP-2**

Zur präparativen Proteolyse von rh-proBMP-2 wurden 50 ml des naturierten Proteins gegen 0,1 M Tris/HCl pH 7,0, 4 M Harnstoff, 1 mM EDTA dialysiert. Aggregate wurden durch Zentrifugation abgetrennt (75.000 g, 1 h) und die Proteinkonzentration spektrophotometrisch bestimmt (1,3 mg/ml). Die Verluste durch die Dialyse betrugen 13 %. Trypsin (Roche Diagnostics) wurde im Verhältnis von 1:100 (Trypsin: proBMP-2; w/w) eingesetzt und der Ansatz 4 h bei 5-7 °C inkubiert. Die Inaktivierung von Trypsin erfolgte mit einem 8-fachen molaren Überschuss an Sojabohnen-Trypsininhibitor (SBTI; Sigma). Die Dialyse des gesamten Ansatzes gegen harnstofffreien Puffer (0,1 M Tris/HCl pH 7,0) fand in Gegenwart von hydrophoben Säulenmaterial (20 ml Fractogel-Phenyl, Merck) statt. Das so mit drBMP-2 beladenene Säulenmaterial wurde in eine Leersäule (XK-16, Pharmacia) gefüllt und anschließend mit:

- 5 SV (Säulenvolumen) Niedrigsalzpuffer (Dialysepuffer),
- 5 SV 50 mM Na-Acetat pH 5,0,
- 2 SV 50 mM Na-Acetat pH 5,0, 4 M Harnstoff und
- 2 SV 50 mM Na-Acetat pH 5,0, 1 M L-Arginin

gewaschen. Eluiert wurde mit 6 M GdmCl, 0,2 M Essigsäure. Die drBMP-2-enthaltenden Fraktionen wurden vereinigt und gegen 0,1 % TFA dialysiert. Es konnte keine Aggregatbildung festgestellt werden. Das Dialysat wurde sterilfiltriert und die Proteinkonzentration spektrophotometrisch bestimmt (1,3 mg/ml). Die Ausbeute an so gewonnenem BMP-2 betrug ca. 50 % bezogen auf das für die limitierte Proteolyse eingesetzte Renaturat. Aus 1 l *E. coli*-Kultur lassen sich somit ca. 17 mg biologisch aktiven drBMP-2s gewinnen bzw. 5,2 (entspricht 5,6 mg reifes BMP-2 incl. N-terminaler Heparinbindestelle), bezogen auf 1 g (Feuchtmasse) induzierter *E. coli*-Zellen. Mit dem von Ruppert et al., [Eur. J. Biochem. 237 (1996), 295--302] beschriebenen Verfahren wurden dagegen nur 0,2 mg BMP-2 je 1 g Zellen erhalten.

**Charakterisierung von drBMP-2**

**Analyse der Proteolyseprodukte durch N-terminale Sequenzierung**

Nach SDS-PAGE und Western-Blot auf eine PVDF-Memran erfolgte die N-terminale Sequenzierung [Edman und Begg, Eur. J. Biochem. 1 (1967), 80-91]. Sie ergab für erfindungsgemäß generiertes drBMP-2 folgende Aminosäuresequenzen:

a) Leu-Lys-Ser-Ser-Cys-Lys-Arg-
b) Lys-Arg-Leu-Lys-Ser-Ser-Cys-
c) Arg-Leu-Lys-Ser-Ser-Cys-Lys-

Das Verhältnis der verschiedenen Spezies zueinander beträgt etwa 3:2:1 (a:b:c). Die Cysteine konnten nicht detektiert werden, jedoch die darauf folgenden Aminosäuren. Die Sequenz wurde jedoch auf DNA-Ebene vollständig verifiziert. Das entspricht dem N-Terminus von reifem BMP-2 ohne die Heparinbindestelle. drBMP-2 wurde in reduzierter und nicht reduzierter Form zur Sequenzierung eingesetzt. Die nicht reduzierte Form konnte lediglich bis zum zweiten Serin sequenziert werden, was dafür spricht, dass der nachfolgende Rest ein Cystin, also Bestandteil einer Disulfidbrücke ist. Insgesamt entspricht somit das Proteolyseprodukt einem N-terminal um 7-9 Aminosäuren verkürzten reifen BMP-2, dem somit die Heparinbindestelle fehlt.

Unter den hier beschriebenen Bedingungen der limitierten Proteolyse werden nicht disulfidverbrückte Peptidketten abgebaut, sodass schließlich nur zu 100 % kovalent dimerisiertes drBMP-2 gewonnen wird.

**Reinheitsanalyse von drBMP-2 mit SDS-Polyacrylamidgelelektrophorese und HPLC**

Die Größe und Reinheit des erfindungsgemäß gewonnenen drBMP-2 wurde mit SDS-PAGE unter reduzierenden und nicht reduzierenden Bedingungen und anschließender Silberfärbung kontrolliert (Bahn 6 und 11 in **Abbildung 4**). Es ist zu 100 % dimerisiert bei einer Reinheit von über 95 %.

Weiterhin wurde zur Überprüfung von Reinheit und Homogenität von drBMP-2 Umkehrphasenhochleistungsflüssigchromatographie (RP-HPLC; Vydac $C_4$) eingesetzt, drBMP-2 eluierte dabei in einem homogenen Peak bei 46,57 % Acetonitril und 0,1 % TFA (**Abbildung 5**), was für die einheitliche Strukturierung und insbesondere Disulfidverbrückung des Proteins spricht.

**Circular-Dichroismus-Spektrometrie**

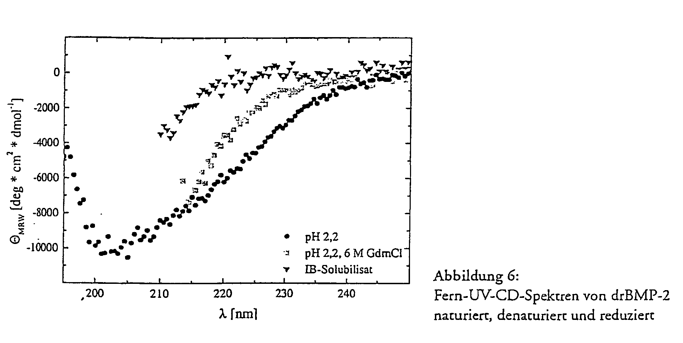

Die starke Differenz zwischen der Elliptizität von naturiertem und denaturiertem drBMP-2 und besonders dem reduzierten und denaturierten reifen BMP-2 (IB-Solubilisat) im Bereich 210-230 nm des Fern-UV-CD-Spektrums (**Abbildung 6**) erlaubt die Feststellung, dass beim erfindungsgemäßen Prozess der Naturierung des Proproteins die Ausbildung von Sekundärstrukturelementen auch in der reifen Domäne des Proteins erfolgt.

# Tabelle 2

Betreff: 3 letter code proBMP (incl. 20 AA His-Tag)

MetGlySerSerHisHisHisHisHisHisSerSerGlyLeuValProArgGlySerHisMetGlyAlaAlaGlyLeuVal
ProGluLeuGlyArgArgLysPheAlaAlaAlaSerSerGlyArgProSerSerGlnProSerAspGluValLeuSerGlu
PheGluLeuArgLeuLeuSerMetPheGlyLeuLysGlnArgProThrProSerArgAspAlaValValProProTyrMet
LeuAspLeuTyrArgArgHisSerGlyGlnProGlySerProAlaProAspHisArgLeuGluArgAlaAlaSerArgAla
AsnThrValArgSerPheHisHisGluGluSerLeuGluGluLeuProGluThrSerGlyLysThrThrArgArgPhePhe
PheAsnLeuSerSerIleProThrGluGluPheIleThrSerAlaGluLeuGlnValPheArgGluGlnMetGlnAspAla
LeuGlyAsnAsnSerSerPheHisHisArgIleAsnIleTyrGluIleIleLysProAlaThrAlaAsnSerLysPhePro
ValThrArgLeuLeuAspThrArgLeuValAsnGlnAsnAlaSerArgTrpGluSerPheAspValThrProAlaValMet
ArgTrpThrAlaGlnGlyHisAlaAsnHisGlyPheValValGluValAlaHisLeuGluGluLysGlnGlyValSerLys
ArgHisValArgIleSerArgSerLeuHisGlnAspGluHisSerTrpSerGlnIleArgProLeuLeuValThrPheGly
HisAspGlyLysGlyHisProLeuHisLysArgGluLysArgGlnAlaLysHisLysGlnArgLysArgLeuLysSerSer
CysLysArgHisProLeuTyrValAspPheSerAspValGlyTrpAsnAspTrpIleValAlaProProGlyTyrHisAla
PheTyrCysHisGlyGluCysProPheProLeuAlaAspHisLeuAsnSerThrAsnHisAlaIleValGlnThrLeuVal
AsnSerValAsnSerLysIleProLysAlaCysCysValProThrGluLeuSerAlaIleSerMetLeuTyrLeuAspGlu
AsnGluLysValValLeuLysAsnTyrGlnAspMetValValGluGlyCysGlyCysArg

**Patentansprüche**

1. Verwendung von Pro-BMP-2 gemäß Tabelle 2 mit der kompletten Pro-Sequenz, die an das reife BMP-2 gebunden ist, zur Herstellung eines Arzneimittels zur Förderung der Knochenheilung und zur Herstellung von Knochenersatzmaterial oder Implantaten.

2. Verwendung nach Anspruch 1 zur Herstellung eines Arzneimittels für spinale Fusionen bei degenerativen Wirbelsäulenerkrankungen, die Regeneration von Schädelknochen, kiefer- und gesichtschirurgische Eingriffe sowie Behandlung komplizierter Knochenfrakturen der Extremitäten.

3. Arzneimittel zur Förderung des Knochenwachstums,
   **gekennzeichnet durch**
   einen Gehalt an Pro-BMP-2 gemäß Tabelle 2 mit der vollständigen Pro-Sequenz Gly[22]-Arg[284] als Wirkstoff.

**4.** Verfahren zur Herstellung eines Arzneimittels zur Förderung des Knochenwachstums,
**dadurch gekennzeichnet,**
**dass** Pro-BMP-2 gemäß Tabelle 2 mit der vollständigen Pro-Sequenz Gly$^{22}$-Arg$^{284}$ in eine zur Verabreichung geeignete Zubereitung formuliert wird.

**5.** Verfahren nach Anspruch 4,
**dadurch gekennzeichnet,**
**dass** die Zubereitung ein physiologisch verträgliches Trägermaterial umfasst, welches mit dem Pro-BMP-2-Wirkstoff beschichtet oder imprägniert ist.

**Claims**

**1.** Use of pro-BMP-2 according to Table 2, having the complete pro sequence which is bonded to the mature BMP-2, for producing a pharmaceutical for promoting bone healing and for producing bone replacement material or implants.

**2.** Use according to Claim 1 in the manufacture of a pharmaceutical for spinal fusions in connection with degenerative diseases of the vertebral column, the neoformation of cranial bones, maxillofacial surgical interventions and treatment of complicated fractures of the bones of the extremities.

**3.** Pharmaceutical for promoting bone growth,
**characterized by**
a content of pro-BMP-2 according to Table 2 having the complete pro sequence Gly$^{22}$-Arg$^{284}$ as active compound.

**4.** Process for producing a pharmaceutical for promoting bone growth,
**characterized in that**
pro-BMP-2 according to Table 2 having the complete pro sequence Gly$^{22}$-Arg$^{284}$ is formulated into a preparation which is suitable for administration.

**5.** Process according to Claim 4,
**characterized in that**
the preparation comprises a physiologically tolerated support material which is coated or impregnated with the pro-BMP-2 active compound.

**Revendications**

**1.** Utilisation de pro-BMP-2 selon le tableau 2 avec la séquence pro complète, qui est liée à la BMP-2 mature, pour la production d'un médicament pour favoriser la cicatrisation osseuse et pour produire des matériaux de substitution osseuse ou des implants.

**2.** Utilisation selon la revendication 1 pour la production d'un médicament pour fusions spinales dans les maladies dégénératives de la colonne vertébrale, la régénération des os crâniens, les interventions chirurgicales maxillaires et faciales ainsi que le traitement des fractures osseuses compliquées des extrémités.

**3.** Médicament pour favoriser la croissance osseuse **caractérisé par** une teneur en pro-BMP-2 selon le tableau 2 avec la séquence pro Gly$^{22}$-Arg$^{284}$ complète comme principe actif.

**4.** Procédé pour produire un médicament pour favoriser la croissance osseuse **caractérisé en ce que** pro-BMP-2 selon le tableau 2 avec la séquence pro Gly$^{22}$-Arg$^{284}$ complète est formulée dans une préparation appropriée à l'administration.

**5.** Procédé selon la revendication 4 **caractérisé en ce que** la préparation comprend un vecteur physiologiquement acceptable qui est revêtu ou imprégné du principe actif pro-BMP-2.

Abbildung 1: SDS-PAGE proBMP-2
1—LMW
2–5—IB-Präparation
6—naturiertes proBMP-2 (n. red.)

Abbildung 2:
Fern-UV-CD-Spektren von proBMP-2
naturiert und denaturiert

Abbildung 3:
Emissionsspektren von proBMP-2
naturiert und denaturiert
($\lambda_{exc}$=280 nm; nicht identische
Proteinkonzentrationen)

Abbildung 4:
SDS-PAGE drBMP-2
1,6—LMW
2–5—drBMP-2, n. red.

Abbildung 5:
RP-HPLC-Chromatogramm drBMP-2

Abbildung 6:
Fern-UV-CD-Spektren von drBMP-2
naturiert, denaturiert und reduziert

Abbildung 7

Abbildung 8

Abbildung 9

Abbildung 10

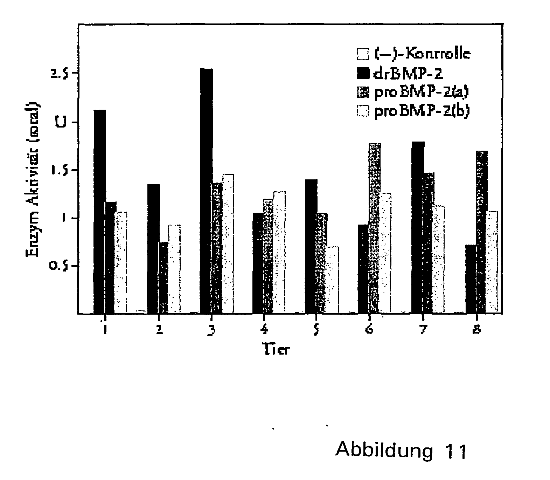

Abbildung 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9855137 A
- US 5013649 A
- US 5674844 A
- US 5650494 A, Cerletti
- US 1997 A
- US 5336602 A, Brinkmann
- US 5593865 A, Rudolph
- WO 0022119 A, Rattenholl

### In der Beschreibung aufgeführte Nicht-Patentliteratur

- **Wozney et al.** *Science,* 1988, vol. 242, 1528-1534
- **Griffith et al.** *Proc. Natl. Acad. Sci. USA,* 1996, vol. 93, 878-883
- **Kaplan et al.** *Calcif. Tissue Int.,* 1990, vol. 47, 117-125
- **Rao et al.** *Hum. Genet.,* 1992, vol. 90, 299-302
- **Shafritz et al.** *N. Engl. J. Med.,* 1996, vol. 335, 555-561
- **Tabas et al.** *Clin. Orthop.,* 1993, vol. 293, 310-316
- **Dudley et al.** *Genes Dev.,* 1995, vol. 9, 2795-2807
- **Zhang ; Bradley.** *Development,* 1996, vol. 122, 2977-2986
- **Lane et al.** *Clin. Orthop.,* 1999, vol. 361, 216-227
- **Marden et al.** *J. Biomed. Mat. Res.,* 1994, vol. 28, 1127-1138
- **Mayer et al.** *Plast. Recontr. Surg.,* 1996, vol. 98, 247-259
- **Sandhu ; Boden.** *Orthop. Clin. North Am.,* 1998, vol. 29, 621-631
- **Toriumi et al.** *Arch. Otolyryngol. Head Neck Surg.,* 1991, vol. 117, 1101-1112
- **Yasko et al.** *J. Bone Joint Surg. (Am.),* 1992, vol. 74, 659-670
- **Boyne et al.** *Int. J. Periodontics Restorative Dent.,* 1997, vol. 17, 11-25
- **Howell et al.** *Int. J. Periodontics Restorative Dent,* 1997, vol. 17, 124-139
- **Israel et al.** *Growth Factors,* 1992, vol. 7, 139-150
- **Wang et al.** *Proc. Natl. Acad. Sci. USA,* 1990, vol. 87, 2220-2224
- **Marston.** *Biochem. J.,* 1986, vol. 240, 1-12
- **Ruppert et al.** *Eur. J. Biochem.,* 1996, vol. 237, 295-302
- **Koenig et al.** *Mol. Cell Biol.,* 1994, vol. 14, 5961-5974
- **Kiefhaber et al.** *Biotechnology (N.Y.),* 1991, vol. 9, 825-829
- **Creighton.** *Biochemistry,* 1997, vol. 378, 731-744
- **Creighton et al.** *Trends Biotechnol.,* 1995, vol. 13, 18-23
- **McDonald ; Hendrickson.** *Cell,* 1993, vol. 73, 421-424
- **Scheufler et al.** *J. Mol. Biol.,* 1999, vol. 287, 103-115
- **Studier ; Moffat.** *J. Mol. Biol.,* 1986, vol. 189, 113-130
- **Sanger et al.** *Proc. Natl. Acad. Sci. USA,* 1997, vol. 74, 5463-5467
- **Brinkmann et al.** *Gene,* 1989, vol. 85, 109-114
- Folding proteins. **Rudolph et al.** Protein Function: A Practical Approach. 1997, 57-99
- **Gill ; Hippel.** *Anal. Biochem.,* 1989, vol. 182, 319-326
- **Edman ; Begg.** *Eur. J. Biochem.,* 1967, vol. 1, 80-91
- Optical spectroscopy to characterize protein conformation and conformational changes. **Schmid.** Protein Structure: A Practical Approach. 1996, 261-297
- **Ruppert et al.** *Eur. J. Biochem.,* 1996, vol. 237, 295-302